(19) Europäisches Patentamt
European Patent Office
Office européen des brevets

(11) Veröffentlichungsnummer: **0 160 946**
**B1**

(12) # EUROPÄISCHE PATENTSCHRIFT

(45) Veröffentlichungstag der Patentschrift: **18.01.89**

(51) Int. Cl.⁴: **C 07 C 45/58**, C 07 C 47/02

(21) Anmeldenummer: **85105415.5**

(22) Anmeldetag: **03.05.85**

(54) **Verfahren zur Herstellung von Aldehyden.**

(30) Priorität: **11.05.84 US 609403**

(43) Veröffentlichungstag der Anmeldung: **13.11.85 Patentblatt 85/46**

(45) Bekanntmachung des Hinweises auf die Patenterteilung: **18.01.89 Patentblatt 89/3**

(84) Benannte Vertragsstaaten: **AT BE CH DE FR GB IT LI NL**

(56) Entgegenhaltungen:
**EP-A- 0 083 130**
**DE-B- 2 252 719**
**US-A- 2 694 090**
**US-A- 3 855 303**

(73) Patentinhaber: **L. GIVAUDAN & CIE Société Anonyme, CH-1214 Vernier-Genève (CH)**

(72) Erfinder: **Naipawer, Richard E., 9 Iris Lane, Wallington, N.J., 07055 (US)**
Erfinder: **Chalk, Alan J., 5 Duchess Drive, Kinnelon, N.J., 07405 (US)**

(74) Vertreter: **Lederer, Franz, Dr. et al, Van der Werth, Lederer & Riederer Patentanwälte Lucile-Grahn-Strasse 22, D-8000 München 80 (DE)**

EP 0 160 946 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Umlagerung von Epoxiden ist eine bekannte Reaktion. Es führt diese bekannte, sauer katalysierte Umlagerung zur Bildung von Carbonylverbindungen, währenddem die basenkatalysierte Umlagerung zur Bildung von Allylalkoholen führt, siehe z. B. J. Org. Chem. 35 (1970), 1598.

Im Falle der sauer katalysierten Umlagerung sind gemäss Schema I 2 Reaktionswege möglich. Gemäss Reaktionsweg A wird die sogenannte innere Expoxidbindung des Ausgangsmaterials gespalten (Bindung 1) und das Wasserstoffatom wandert vom terminalen C-Atom zum internen C-Atom, wobei der Aldehyd 1 gebildet wird. Gemäss Reaktionsweg B wird die äussere Epoxidbindung gespalten (Bindung 2) und der Rest $R_2$ wandert vom inneren C-Atom zum End-C-Atom, wobei Keton 2 resultiert. Im Falle von $R_2$=H handelt es sich beim Produkt um ein Methylketon, also ein Molekül, das dasselbe Kohlenstoffgerüst wie das reagierende Epoxid aufweist. Andernfalls, d.h. wenn $R_2$ einen kohlenwasserstoffhaltigen Substituenten trägt, weist das resultierende Keton ein ungelagertes Kohlenwasserstoffskelett auf.

Schema I

Reaktionsweg a

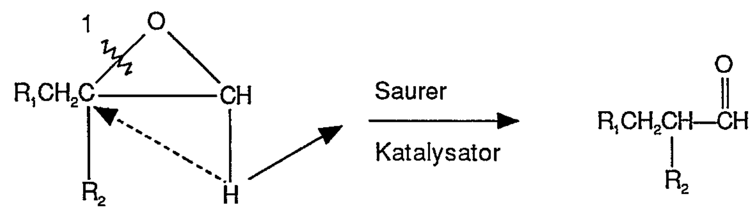


1

Reaktionsweg b

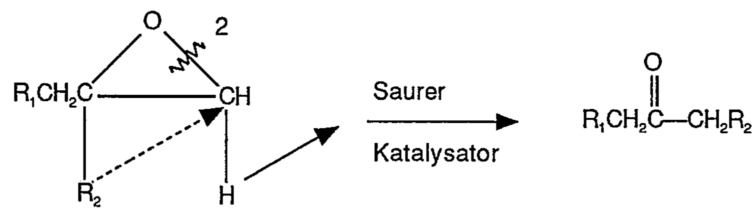


2

Die Natur des $R_2$-Substituenten - seine sterischen Effekte, bedingt durch Umfang des Restes, seine induktiven und seine repulsiven elektronischen Effekte, die inhärenten Charakteristiken des involvierten Epoxids (Ringspannung, Dipol-Dipol-Zwischenwirkungen, etc.) und die verschiedenen Eigenschaften des sauren Katalysators werden selbstverständlicherweise ebenfalls einen Einfluss darauf ausüben, welche Reaktion im einzelnen Fall abläuft, und auf das Verhältnis von Aldehyd und Keton im Reaktionsprodukt.

Im Falle eines 1,2-Epoxides mit $R_2$=H - und demzufolge minimalen sterischen und elektronischen Effekten ist es schwierig, Reaktionsbedingungen zu finden, die dazu führen, dass entweder exklusiv Reaktion A oder B abläuft. Im Falle der bekannten Methoden unter Verwendung von Katalysatoren wie Mineralsäuren oder Lewis-Säuren resultieren üblicherweise Gemische von Aldehyden und Ketonen.

Aus der US-PS 3 855 303 ist die Umlagerung von terminalen Epoxiden zu den entsprechenden Aldehyden, enthaltend eine geringe Menge des entsprechenden Methylketons, bekannt geworden. Das Verfahren benützt Alkalimetallperchlorate in Kombinationen mit Trialkyl- und Triarylphosphinoxiden als Katalysatoren zur Umlagerung. Die technische und kommerzielle Zweckmässigkeit dieses Verfahrens ist recht fragwürdig: Es werden keine Angaben zu den Ausbeuten gemacht und gemäss dem einzigen Beispiel dieser Patentschrift wird eine Reaktionszeit von 72 Stunden angegeben. Dieses Verfahren wurde in der Folge wiederholt, wobei eine Reaktionszeit von 72 Stunden und eine Reaktionstemperatur von 140°C eingehalten wurde. Es wurde nun allerdings ein hohes Aldehyd/Ketonverhältnis gefunden, wie dies auch aus der Literaturstelle hervorgeht, jedoch war die Ausbeute sehr gering, nämlich nur 19%.

Organoleptisch reine n-Aldehyde, wie n-Hexanal, n-Heptanal, n-Decanal, n-Dodecanal, etc., sind wichtige und sehr nützliche Ingredienzien für Geschmackstoff- und Riechstoffkompositionen, siehe z.B. S. Arctander, «Perfume und Flavor Chemicals», Band I und II, Steffen Arctander, Publisher, Montclair, N.J. (1969). Es gibt nun kein zur Herstellung solcher Aldehyde geeignetes Verfahren ausgehend aus terminalen Epoxiden, bei dem nicht gleichzeitig beträchtliche Mengen von Ketonen gebildet würden (siehe z.B. Rickborn, et al., J. Amer. Chem. Doc. 93, (1971), 1693-1700), oder wobei nicht Reaktionsbedingungen eingehalten werden müssten, die kommerziell unattraktiv sind, eben beispielsweise solche, die oben besprochen sind. Da es weiterhin bekannt ist, dass terminale Epoxide ökonomisch aus terminalen Olefinen hergestellt werden können, welche letztere leicht und billig in der Petroliumindustrie anfallen, bestand ein Bedürfnis für ein ökonomisches und kommerziell attraktives Verfahren zur Überführung solcher Epoxide in Aldehyde, und zwar für ein solches Verfahren, bei dem nicht noch gleichzeitig substantielle Mengen an ungewünschten Ketonen anfallen.

Die vorliegende Erfindung betrifft nun ein Verfahren zur Herstellung von Aldehyden der Formel $R-CH_2-CHO$, worin R ein Rest ist, der unter den unten genannten Reaktionsbedingungen stabil ist, welches Verfahren dadurch gekennzeichnet ist, dass man das entsprechende terminale Epoxid in Anwesenheit einer katalytischen Menge von Lithiumtetrafluorborat und in Anwesenheit eines Polyäthers als Lösungsmittel durchführt. Der zu verwendende Polyäther wird unten genauer detailliert. Die gewünschten Aldehyde fallen in guter Ausbeute an und mit dem Vorteil, dass nicht noch gleichzeitig beträchtliche Mengen an Ketonen als Nebenprodukte anfallen.

Genauer gesagt, wird das 1,2-Epoxid durch Erhitzen auf eine Temperatur zwischen 80°C und 200°C im Lithiumtetrafluoroborat-Polyäthersystem isomerisiert, wobei der Aldehyd mit einem günstigen Alkanal/Alkan-2-on-Verhältnis anfällt.

Es ist höchstens überraschend, dass 1,2-Epoxide mittels Lithiumtetrafluorborat selektiv und in guten Ausbeuten zu Aldehyden umgelagert werden können, wenn man davon ausgeht, dass Lithiumtetrafluoroborat üblicherweise als Mittel für die cyclische Oligomerisierung von Äthylenoxid verwendet wird, siehe z.B. J. Dale und K. Daasvatn, J. Chem. Soc., Chem. Commun., (1976) (8), 295.

Das erfindungsmässige Verfahren kann wie folgt illustriert werden:

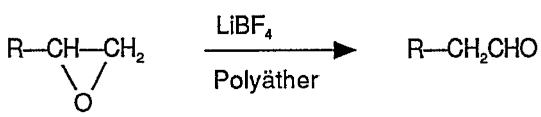


II I

worin R die obige Bedeutung aufweist.

Die Erfindung beinhaltet die Umlagerung von Epoxiden zu den entsprechenden Aldehyden; die Natur des Restes R ist nicht kritisch, solange ein solcher Rest R eingesetzt wird, der unter den Reaktionsbedingungen stabil ist.

Das erfindungsgemässe Verfahren wird aber vorzugsweise auf solche Ausgangsmaterialien II angewendet, worin R ein Kohlenwasserstoffrest ist, vorzugsweise ein Kohlenwasserstoffrest mit drei C-Atomen oder mehr. Obschon das Verfahren auch mit R=Methyl oder Äthyl funktioniert, ist doch der Siedepunkt der entsprechenden Ausgangsmaterialien geringer als die vorliegende Reaktionstemperatur und es müssten demgemäss Druckkessel eingesetzt werden, um unter den optimalen Reaktionstemperaturen arbeiten zu können. Es existiert keine maximale Grenze für die Grösse der R-Reste, aber aus praktischen Erwägungen sind Epoxide mit R=18 oder weniger C-Atomen bevorzugt, da die Olefine, d.h. die Ausgangsmaterialien für diese höheren Epoxide üblicherweise weniger leicht erhältlich sind. Speziell bevorzugt wird das Verfahren zur Herstellung von Aldehyden mit R=4–12 C-Atomen eingesetzt, da diese Aldehyde die grösste kommerzielle Bedeutung aufweisen. R kann gerade oder verzweigt sein, jedoch sind die geradkettigen Reste bevorzugt, da die entsprechenden Aldehyde kommerziell wichtiger sind.

Wie oben gesagt, wird gemäss dem vorliegenden Verfahren das Epoxid in Abwesenheit von Lithiumtetrafluoroborat und einem Polyäther als Lösungsmittel zwischen 80°C bis 200°C zur Reaktion gebracht. Obschon das Verfahren unter Benützung verschiedener Reaktionsvarianten erfolgreich durchgeführt werden kann, hat es sich doch herausgestellt, dass gewisse Parameter bevorzugt sind und zwar darum, weil sie die besten Ausbeuten erbringen.

So wird Lithiumtetrafluoroborat vorzugsweise mit dem Polyäther gemischt, das Gemisch auf die erfindungsgemässe definierte Reaktionstemperatur erhitzt und das umzusetzende Epoxid über eine gewisse Zeit zu dem Gemisch zugegeben. Es wird vermutet, dass die hauptsächlichste Nebenreaktion in einer gewissen Polymerisation des Epoxides besteht. Beim vorsichtigen, nicht allzu raschen Zugeben des Epoxides zum Reaktionsgemisch tritt diese Nebenreaktion erfahrungsgemäss im geringsten Umfang ein. Dabei entstehen die Aldehyde in diesem Fall auch mit besten Ausbeuten. Die zweckmässige Reaktionszeit kann z.B. 0 bis 24 Stunden betragen. Aus praktischen Erwägungen genügt eine Reaktionszeit von 6 Stunden in den allermeisten Fälllen, wobei eine Reaktionszeit von 2 bis 4 Stunden bevorzugt ist. Natürlich hängt die optimale Zufuhr auch von der Grösse der Charge ab, der Konzentration von weiteren Reagenzien und der Reaktionszeit. Die Bestimmung der optimalen Zufuhr ist aber reines fachmännisches Wissen.

Aufgrund der obigen Angaben ist ersichtlich, dass im Vordergrund des Interesses ein Verfahren zur Herstellung von Aldehyden der Formel

$R-CH_2CHO$ I

worin R ein Alkylrest mit 3–18 Kohlenstoffatomen ist, steht, welches Verfahren dadurch gekennzeichnet ist, dass man ein Epoxid der Formel

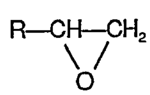 II

worin R obige Bedeutung besitzt, bei einer Temperatur zwischen 80°C und 200°C zu einem Gemisch von Lithiumtetrafluoroborat und einem Polyäther, welch letzterer in der 1- bis 20fachen Gewichtsmenge des Epoxids vorliegt, gibt und aus dem Reaktionsgemisch den gebildeten Aldehyd isoliert.

Die Menge des eingesetzten Lithiumtetrafluoroborats ist nicht kritisch, zweckmässigerweise werden aber mindestens 0,1 Gewichtsprozent, berechnet auf das Gewicht des eingesetzten Epoxides, eingesetzt. Mengen von >5 Gewichtsprozent bringen keinen Vorteil mehr. Aus praktischen Erwägungen sind Mengen von 1–2 Gewichtsprozent bevorzugt, wobei Mengen von 1,5–1,8 Gewichtsprozent die speziell bevorzugten Mengen darstellen.

Die Anwesenheit eines Polyäthers ist wesentlich, nicht aber dessen Volumen. Es wird vermutet, dass der Polyäther dazu dient, das Lithiumion des Katalysators zu solvatisieren, das Anion des Katalysators steht so besser zur Verfügung, eine Beobachtung, die ja auch mit Kronen-Äthern gemacht wurde. Zu diesem Zweck ist es erforderlich, genügend Polyäther einzusetzen. Aus praktischen Erwägungen heraus wird deshalb der Polyäther vorzugsweise als Lösungsmittel eingesetzt, wobei mindestens 1 g Lösungsmittel pro g Epoxid eingesetzt wird. Es hat sich gezeigt, dass keine Vorteile entstehen, wenn über 20 g Polyäther pro g Epoxid eingesetzt wird; ein bevorzugter Bereich ist demzufolge der von 8:1–2:1, wobei 3:1–5:1 ein speziell bevorzugter Bereich darstellt.

Die Natur des Polyäthers ist nicht kritisch. Es kann irgendeine polyoxygenerierte Spezies, wie beispielsweise ein Dialkyläther von Glykolen und Dialkyläther von Polyglykolen als auch solche makrocyclischen Polyäther, die unter dem Namen Kronen-Äther bekannt geworden sind, als Lösungsmittel zum Einsatz gelangen.

Aus praktischen Erwägungen heraus wird man die kommerziell leicht erhältlichen Dialkyläther von Äthylenglykol und Polyäthylenglykolen einsetzen, wobei hier Glym (Äthylenglykoldimethyläther), Diglym (Diäthylenglykoldimethyläther) und Tetraglym (Tetraäthylenglykoldimethyläther) besonders bevorzugt sind. Im Vordergrund des Interesses steht insbesondere Tetraglym.

Auf Grund obiger Angaben kann demgemäss der Polyäther zweckmässigerweise als Verbindung der Formel

$R'O-(CH_2-[CH_2]_n-O-)_pR''$ definiert werden, worin $n=1-2$, $p=1, 2, 3, 4, 5$ oder 6 und R' und R'' gleich oder verschieden sind und Alkylreste mit 1–4 Kohlenwasserstoffatomen darstellen. Vorzugsweise ist $R=R''=$Methyl, $n=1$ und $p=2-5$, insbesondere $p=4$.

Wie zu erwarten war, beeinflusst die Temperatur sowohl Geschwindigkeit des Ablaufs als auch Selektivität der Reaktion im Sinn, dass, je höher die Temperatur, je rascher der Umsatz, aber gleichzeitig die Reaktion weniger selektiv ist. Aus praktischen Gründen ist deshalb unterhalb 80°C die Reaktionsgeschwindigkeit zu gering und oberhalb 200°C ist die Selektivität zu gering. Es hat sich herausgestellt, dass ein Temperaturbereich von 90°C bis 150°C bevorzugt ist, wobei im Vordergrund des Interesses ein Temperaturbereich von 110°C bis 140°C steht.

Es hat sich weiter herausgestellt, dass auch noch weitere Parameter das erfindungsgemässe Verfahren in vorteilhafter Weise beeinflussen. Das Einhalten dieser zusätzlichen Parameter ist jedoch nicht zwingend. So können beispielsweise zur Minimalisierung von Autoxidation des Aldehydes ein Antioxidans, wie beispielsweise butyliertes Hydroxytoluol (BHT), butyliertes Hydroxyanisol (BHA) oder Hydrochinon (HQ) etc., zugegeben werden und zwar zweckmässigerweise in Mengen von 0,1–0,5 Gewichtsprozent. Zusätzlich kann die Reaktion gegebenenfalls in einer inerten Gasatmosphäre durchgeführt werden, ebenfalls zur Verhinderung von Autoxidation. Als solche Gase kommen Stickstoff, Argon, etc. in Frage.

Ein weiterer Vorteil ergibt sich durch die Zugabe von Lithiumcarbonat. Es wird vermutet, dass das Lithiumcarbonat dazu dient, allenfalls vorhandene Säure zu neutralisieren. Aus diesem Grund hat denn auch Lithiumcarbonat einen positiven Einfluss auf die Ausbeute, nicht aber auf das Verhältnis von Aldehyd zu Keton.

Beispiele

Die folgenden Beispiele illustrieren die praktische Durchführung des erfindungsgemässen Verfahrens, sollen aber nicht einschränkend verstanden werden. Zur Charakterisierung der Reaktionsprodukte bzw. der Verbindungen in den Reaktionsprodukten dienten u.a.:

1. IR-Spektroskopie, unter Verwendung eines Perkin-Elmers Modell 137; Verbindungen jeweils ohne Zusatz von Lösungsmitteln untersucht.
2. Gaschromatographie: Kolonne bzw. Kapillarkolonne, enthaltend 10% Carbowax 20M (¼ inch I.D. × 6 Fuss [1 inch = 2,54 cm, 1 Fuss = 39,48 cm]) bzw. enthaltend geschmolzenen/gesinterten Quarzsand/Silicon-Öl SE-30 (0,25 mm I.D. × 30 Meter).

Allgemeines Verfahren zur Umlagerung der Epoxide

Das Lithiumtetrafluoroborat und gegebenenfalls ein Antioxidans wie oben definiert werden in das Reaktionsgefäss gegeben, das bereits den Polyäther als Lösungsmittel enthält. Gegebenenfalls wird das Reaktionsgefäss mit einem inerten Gas gespült und diese Spülung während der ganzen Reaktionszeit aufrecht erhalten.

Das resultierende Gemisch wird unter Rühren auf die gewünschte Reaktionstemperatur erhitzt. Hierauf wird das entsprechende Epoxid während einer gewissen Zeit zu diesem Gemisch zugegeben.

Der Verlauf der Umlagerung kann verfolgt werden, indem in gewissen Zeitabständen ein Aliquot, beispielsweise 0,5–1 ml, dem Reaktionsgemisch entnommen wird, dieser Aliquot zu Wasser gegeben wird (z.B. 5–10 ml) und hierauf die obere (die ölige Phase) gaschromatographisch analysiert wird. Rühren und Erhitzen des Gemisches werden zweckmässig solange fortgesetzt, bis eine gaschromatische Analyse ergibt, dass nur noch 1% des Ausgangsmaterials oder noch weniger vorhanden ist. Das Reaktionsgemisch wird hierauf auf die Umgebungstemperatur abgekühlt und zweckmässigerweise zu einem äquivalenten Volumen Wasser gegeben. Man kann nun das resultierende Gemisch in an sich bekannter Weise mit Hexan oder anderen geeigneten Solventien extrahieren und den erhaltenen Extrakt mit Wasser zurückwaschen, um allenfalls mitgeschleppten Polyäther zu entfernen.

Der Extrakt wird konzentriert und das Produkt durch Destillieren, zweckmässigerweise unter Vakuum, isoliert. Die Ergebnisse der Beispiele 1–11 illustrierten dieses Verfahren.

Tabelle I

| Beispiel | Epoxid | Lösungs-mittel[a] | Gewichts-% LiBF$_4$ | Gewichts-% Li$_2$CO$_3$[b] | Reaktions-temperatur | Zeit (h) Epoxid-zugabe | Zeit (h) Zusätzliche Reaktions-dauer | Aldehyd | Ausbeute %[c] | Alkanal Alkan-2-ON[d] |
|---|---|---|---|---|---|---|---|---|---|---|
| 1 | 1,2-Epoxydecan (46,8 g) | Tetraglym | 2,1 | 0,0 | 140° | 0,5 | 0,75 | n-Decanal | 38,9 | 17,4/1,0 |
| 2 | 1,2-Epoxydecan (46,8 g) | Tetraglym | 1,6 | 0,0 | 120° | 1,5 | 2,25 | n-Decanal | 46,8 | 23,1/1,0 |
| 3 | 1,2-Epoxydecan (46,8 g) | Tetraglym | 1,6 | 0,0 | 120° | 3,0 | 1,25 | n-Decanal | 53,4 | 25,8/1,0 |
| 4 | 1,2-Epoxydecan (46,8 g) | Tetraglym | 1,6 | 0,2 | 120° | 1,5 | 2,25 | n-Decanal | 51,1 | 24,8/1,0 |
| 5 | 1,2-Epoxydecan (46,8 g) | Tetraglym | 1,6 | 0,2 | 120° | 3,0 | 1,75 | n-Decanal | 57,1 | 25,6/1,0 |
| 6 | 1,2-Epoxydecan (46,8 g) | Glym | 1,6 | 0,2 | 90° | 3,0 | 3,25 | n-Decanal | 35,7 | 29,3/1,0 |
| 7 | 1,2-Epoxydecan (46,8 g) | Tetraglym | 2,2 | 0,4 | 140° | 0,5 | 1,0 | n-Decanal | 45,8 | 18,5/1,0 |
| 8 | 1,2-Epoxydecan (46,8 g) | Teraglym | 2,0 | 0,5 | 120° | 3,0 | 0,5 | n-Decanal | 49,4 | 22,8/1,0 |
| 9 | 1,2-Epoxydodecan (55,2 g) | Tetraglym | 1,7 | 0,5 | 120° | 3,0 | 0,5 | n-Dodecanal | 48,7 | 22,9/1,0 |
| 10 | 1,2-Epoxytetradecan (63,6 g) | Tetraglym | 1,5 | 0,4 | 120° | 3,0 | 0,5 | n-Tetradecanal | 48,4 | 26,1/1,0 |
| 11[e] | 1,2-Epoxydecan (46,8 g) | Tetraglym | 1,6 | 0,0 | 120° | 0,5 | 2,25 | n-Decanal | 34,0 | 10,8/1,0 |

[a] Volumen, 200 ml; Tetraglym = Tetraäthylen-glycol-dimethyl-äther; Glym = Äthylen-glycol-dimethyl-äther
[b] Lithiumcarbonat als Buffer zugegeben
[c] Ausbeute: in % des eingesetzten Epoxids angeben
[d] erhalten durch GLC: 0,25 mm (I.D.) × 30 Meter/SiO$_2$-Siliconöl SE-30 Kapillarkolonne; Temperaturprogramm 100°→ 200 °C/10 °/Min.
[e] Triphenylphosphinoxid (Ph$_3$PO) – Zugabe, 1,6 Gew.-%

Aus der Tabelle ist ersichtlich, dass in den Beispielen 4-10 Lithiumcarbonat als Puffer verwendet wurde, und zwar Lithiumcarbonat in Mengen von 0,2-0,5 Gewichtsprozent. Die Zugabe dieses Lithiumcarbonats in Mengen von bis zu 5 Gewichtsprozent ist demgemäss vorteilhaft, aber nicht wesentlich für das erfindungsgemässe Verfahren.

Aus Beispiel 11 ist der Effekt von Triphenylphosphinoxid auf das Reaktionssystem ersichtlich. Es ist bekannt, dass dieses Oxid ein kräftiger Co-Katalysator ist, er hat jedoch einen negativen Effekt auf das Verhältnis Aldehyd/Keton (Beispiele 2 und 11).

**Patentansprüche**

1. Verfahren zur Herstellung von Aldehyden der Formel

$R-CH_2CHO$ I

worin R $C_{3-18}$-Alkyl darstellt, dadurch gekennzeichnet, dass man bei Temperaturen von 80 °C bis 200 °C ein Epoxid der Formel

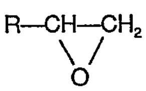

II

worin R obige Bedeutung besitzt, zu einem Gemisch von Lithiumtetrafluoroborat und einem Polyäther, welch letzterer in der 1- bis 20fachen Gewichtsmenge des Epoxids vorliegt, zugibt und aus dem Reaktionsgemisch den erhaltenen Aldehyd I isoliert.

2. Verfahren gemäss Anspruch 1, dadurch gekennzeichnet, dass R einen $C_{4-12}$-Alkylrest darstellt.

3. Verfahren nach Anspruch 1 oder 2, dadurch gekennzeichnet, dass R ein geradkettiger Alkylrest ist.

4. Verfahren nach einem der Ansprüche 1-3, dadurch gekennzeichnet, dass das Epoxid innerhalb von 6 Stunden zum Reaktionsgemisch zugegeben wird.

5. Verfahren nach einem der Ansprüche 1-4, dadurch gekennzeichnet, dass die Menge des verwendeten Lithiumtetrafluoroborats 0,5-5 Gewichtsprozent, bezogen auf das eingesetzte Epoxid, beträgt.

6. Verfahren nach einem der Ansprüche 1-5, dadurch gekennzeichnet, dass die Menge des Lithiumtetrafluoroborats zwischen 1 und 2 Gewichtsprozent beträgt.

7. Verfahren nach einem der Ansprüche 1-6, dadurch gekennzeichnet, dass die Menge des Lithiumtetrafluoroborats zwischen 1,5 und 1,8 Gewichtsprozent beträgt.

8. Verfahren nach einem der Ansprüche 1-7, dadurch gekennzeichnet, dass der benützte Polyäther ein Dialkyläther eines Glykols oder ein Dialkyläther eines Polyglykols oder einen Kronen-Äther darstellt.

9. Verfahren nach einem der Ansprüche 1-8, dadurch gekennzeichnet, dass der Polyäther die Formel $R'O-(CH_2-[CH_2]_n-O-)_pR''$ aufweist, worin R' und R'' gleich oder verschieden sind und Alkylreste mit 1-4 Kohlenstoffatomen darstellen, n 1 oder 2 ist und p 1-6 ist.

10. Verfahren nach einem der Ansprüche 1-9, dadurch gekennzeichnet, dass der Polyäther eine Verbindung der Formel $CH_3-O-(CH_2-CH_2-O-)_p CH_3$ darstellt, worin p 1-4 ist.

11. Verfahren nach einem der Ansprüche 1-10, dadurch gekennzeichnet, dass man den Polyäther in der 2-8fachen Menge des Epoxides einsetzt.

12. Verfahren nach einem der Ansprüche 1-11, dadurch gekennzeichnet, dass man zwischen 90 °C und 150 °C arbeitet.

13. Verfahren nach einem der Ansprüche 1-12, dadurch gekennzeichnet, dass die Reaktionstemperatur zwischen 110 °C und 140 °C liegt.

**Claims**

1. A process for the manufacture of aldehydes of the formula

$R-CH_2CHO$ I

wherein R represents $C_{3-18}$-alkyl, characterized by adding an epoxide of the formula

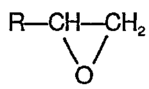

II

wherein R has the above significance, at temperatures of 80 °C to 200 °C to a mixture of lithium tetrafluoroborate and a polyether, which latter is present in a 1- to 20-fold amount by weight of the epoxide, and isolating the aldehyde I obtained from the reaction mixture.

2. A process according to claim 1, characterized in that R represents a $C_{4-12}$ alkyl residue.

3. A process according to claim 1 or 2, characterized in that R is a straight-chain alkyl residue.

4. A process according to any one of claims 1-3, characterized in that the epoxide is added to the reaction mixture within 6 hours.

5. A process according to any one of claims 1-4, characterized in that the amount of lithium tetrafluoroborate used is 0.1-5 weight percent based on the epoxide used.

6. A process according to any one of claims 1-5, characterized in that the amount of lithium tetrafluoroborate is between 1 and 2 weight percent.

7. A process according to any one of claims 1-6, characterized in that the amount of lithium tetrafluoroborate is between 1.5 and 1.8 weight percent.

8. A process according to any one of claims 1-7, characterized in that the polyether used is a dialkyl ether of a glycol or a dialkyl ether of polyglycol or a crown ether.

9. A process according to any one of claims 1-8, characterized in that the polyether has the

formula $R'O-(CH_2-[CH_2]_n-O-)_pR''$ in which R' and R'' are the same or different and represent alkyl residues with 1-4 carbon atoms, n is 1 or 2 and p is 1-6.

10. A process according to any one of claims 1-9, characterized in that the polyether is a compound of the formula $CH_3-O-(CH_2-CH_2-O-)_pCH_3$ in which p is 1-4.

11. A process according to any one of claims 1-10, characterized in that the polyether is used in a 2-8 fold amount of the epoxide.

12. A process according to any one of claims 1-11, characterized in that one works between 90 °C and 150 °C.

13. A process according to any one of claims 1-12, characterized in that the reaction temperature lies between 110 °C and 140 °C.

## Revendications

1. Procédé de préparation d'aldéhydes de formule

$$R-CH_2CHO \qquad I$$

dans laquelle R représente un alkyl $C_{3-18}$, caractérisé en ce que, à des températures de 80 °C à 200 °C on ajoute un époxyde de formule

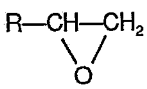
II

dans laquelle R a la signification ci-dessus, à un mélange de tétrafluoroborate de lithium et de polyéther, ce dernier représentant une quantité 1 à 20 fois en poids de celle de l'époxyde et on isole du mélange réactionnel l'aldéhyde I obtenu.

2. Procédé selon la revendication 1, caractérisé en ce que R représente un alcoyle $C_{4-12}$.

3. Procédé selon l'une des revendications 1 ou 2, caractérisé en ce que R est un alcoyle linéaire.

4. Procédé selon l'une des revendications 1-3, caractérisé en ce que l'époxyde est ajouté au mélange réactionnel en l'espace de 6 heures.

5. Procédé selon l'une des revendications 1-4, caractérisé en ce que la quantité de tétrafluoroborate de lithium utilisée représente 0,1-5 pour cent en poids rapporté à l'époxyde mis en œuvre.

6. Procédé selon l'une des revendications 1-5 caractérisé en ce que la quantité de tétrafluoroborate de lithium est comprise entre 1 et 2 pour cent en poids.

7. Procédé selon l'une des revendications 1-6, caractérisé en ce que la quantité de tétrafluoroborate de lithium est comprise entre 1,5 et 1,8 pour cent en poids.

8. Procédé selon l'une des revendications 1-7, caractérisé en ce que le polyéther utilisé est un dialkyléther d'un glycol ou un dialkyléther d'un polyglycol ou d'un éther-couronne.

9. Procédé selon l'une des revendications 1-8, caractérisé en ce que le polyéther présente la formule $R'O-(CH_2-[CH_2]_n-O-)_pR''$ dans laquelle R' et R'' sont identiques ou différents et représentent des alcoyles avec 1-4 atomes de carbone, n est 1 ou 2 et p est 1 à 6.

10. Procédé selon l'une des revendications 1-9, caractérisé en ce que le polyéther est un composé de formule $CH_3-O-(CH_2-CH_2-O-)_pCH_3$, dans laquelle p est 1-4.

11. Procédé selon l'une des revendications 1-10, caracterisé en ce qu'on utilise le polyéther en une quantité 2 à 8 fois supérieure à celle de l'époxyde.

12. Procédé selon l'une des revendications 1-12, caractérisé en ce qu'on travaille entre 90 °C et 150 °C.

13. Procédé selon l'une des revendications 1-12, caractérisé en ce que la température de réaction est comprise entre 110 °C et 140 °C.